# EUROPEAN PATENT SPECIFICATION

(11) **EP 2 201 376 B1**
(45) Date of publication and mention of the grant of the patent: **08.08.2012**
(21) Application number: 08842581.4
(22) Date of filing: 15.10.2008
(51) Int. Cl.: G01N 33/573, C07K 14/705, C12N 9/16, G01N 33/574

(54) **A NEW BIOMARKER FOR MONITORING DEVELOPMENT OF DISEASES AND ASSESSING THE EFFICACY OF THERAPIES**
NEUER BIOMARKER ZUR ÜBERWACHUNG DER ENTWICKLUNG VON KRANKHEITEN UND ZUM TESTEN DER WIRKSAMKEIT VON THERAPIEN
NOUVEAU BIOMARQUEUR POUR SURVEILLER LE DÉVELOPPEMENT DE MALADIES ET ÉVALUER L'EFFICACITÉ DE THÉRAPIES

(30) Priority: 24.10.2007 FI 20070795
(43) Date of publication of application: 30.06.2010
(60) Divisional application: 12002502.8
(73) Proprietor: Faron Pharmaceuticals OY, 20520 Turku (FI)
(72) Inventor: JALKANEN, Sirpa, FI-20760 Piispanristi (FI); SALMI, Marko, FI-20900 Turku (FI); JALKANEN, Markku, FI-20760 Piispanristi (FI)
(74) Representative: Öhman, Ann-Marie
(86) International application number: PCT/FI2008/050576
(87) International publication number: WO 2009/053523

(56) References cited:
- WO-A1-2004/079013
- WO-A1-2004/084933
- WO-A1-2007/107598
- WO-A2-2007/025044
- US-A1- 2006 198 821
- "OPL121 IFN-beta-treatment augments adenosine production via CD73 (ecto-5'-nucleotidase) upregulation both on cultured endothelial cells in vitro and in multiple sclerosis patients in vivo", JOURNAL OF NEUROLOGICAL SCIENCES, ELSEVIER SCIENTIFIC PUBLISHING CO, AMSTERDAM, NL, vol. 238, 1 January 2005 (2005-01-01), page S77, XP005546120, ISSN: 0022-510X
- JOHNSON, S.M. ET AL.: '5'-Nucleotidase as a marker of both general and local inflammation in rheumatoid arthritis patients.' RHEUMATOLOGY vol. 38, no. 5, 1999, pages 391 - 396, XP008131941
- ECKLE, T. ET AL.: 'Identification of ectonucleotidases CD39 and CD73 in innate protection during acute lung injury.' JOURNAL OF IMMUNOLOGY vol. 178, no. 12, 2007, pages 8127 - 8137, XP008131940
- AIRAS, L. ET AL.: 'Mechanism of action of IFN-beta in the treatment of multiple sclerosis: a special reference to CD73 and adenosine.' ANNALS OF THE NEW YORK ACADEMY OF SCIENCES vol. 1110, September 2007, pages 641 - 648, XP008131944
- THOMSON, L.F. ET AL.: 'Production and characterization of monoclonal antibodies to the glycosyl phosphatidylinositol-anchored lymphocyte differentiation antigen ecto-5'-nucleotidase (CD73).' TISSUE ANTIGENS vol. 35, no. 1, 1990, pages 9 - 19, XP008131950
- UEDA, Y. ET AL.: 'Pravastatin restored the infarct size-limiting effect of ischemic preconditioning blunted by hypercholesterolemia in the rabbit model of myocardial infarction' JOURNAL OF THE AMERICAN COLLEGE OF CARDIOLOGY vol. 34, no. 7, 1999, pages 2120 - 2125, XP008131946
- ZHOU, X. ET AL.: 'Effects of ecto-5'-nucleotidase on human breast cancer cell growth in vitro and in vivo.' ONCOLOGY REPORTS vol. 17, no. 6, 2007, pages 1341 - 1346, XP008131902

## Description

### FIELD OF THE INVENTION

This invention concerns the use of CD73 in tissue fluids as a biomarker for monitoring development of diseases. Also methods for determining CD73 protein in a tissue fluid are described.

### BACKGROUND OF THE INVENTION

The publications and other materials used herein to illuminate the background of the invention, and in particular, cases to provide additional details respecting the practice, are incorporated by reference.

CD73 is a cell-surface enzyme that has 5'-ectonucleotidase activity. It thus mediates the conversion of monophosphorylated purine nucleotides into corresponding nucleosides. For example, dephosphorylation of AMP to adenosine is catalyzed by CD73. CD73 is also present as a soluble form in the plasma and the soluble enzyme has the same enzymatic activity as the membrane bound form.

Adenosine is one of the physiological regulators of endothelial cell permeability [1, 2], and can thus be involved in the pathogenesis of many disorders like acute lung injury, systemic inflammatory response syndrome, respiratory distress syndrome, high-altitude sickness. Changes in endothelial permeability also take place in inflammation, in traumas and in cancer. CD73 controls endothelial permeability via an adenosine-mediated mechanism in normal conditions, hypoxia and ventilator-induced lung injury [3-7].

CD73 is induced by certain cytokines. Most importantly, interferon alpha and beta have been reported to increase the expression and activity of CD73 in humans (8, WO 2004/084933, 11). These cytokines are also clinically used to treat different diseases. Interferon alpha, for instance is used to treat certain infections and malignancies such as hepatitis and hairy cell leukemia. Interferon beta, on the other hand, is widely used to dampen inflammation in multiple sclerosis. However, in many cases the beneficial response to interferon treatment is only seen in a subpopulation of patients, and also the initially responding patients can later became refractory to the treatment. Thus, there is a need to develop easily measurable biomarkers that reflect the biological responsiveness of the body to the treatment.

The patent publication WO 2004/084933 discloses the use of cytokines for inducing endothelial CD73 expression and subsequently elevating the adenosine level in an individual. The use of interferon beta in combination with adenosine monophosphate (AMP) in the treatment of multi-organ failure in rats is described.

The patent publication WO 2007/042602 describes the use of plain interferon beta for treatment or prevention of ischemia reperfusion injury or multi-organ failure.

Statins, hypolipidemic agents used to lower cholesterol level, are known to induce CD73 expression in the patients.

Aso known is the use of CD73 as a marker of both general and local inflammation in rheumatoid artritis [12].

However, there is no disclosure in prior art concerning measuring CD73 protein in serum or any other tissue fluid for use as a biomarker for monitoring development of diseases or for assessment of efficacy of therapies.

### SUMMARY OF THE INVENTION

We have shown that measurement of soluble CD73 activity can be used to monitor disease severity and responsiveness to the therapy. Therefore, we believe that analysis of CD73 expression level or activity by any technique may provide valuable information about the course of a disease or treatment response.

The present invention concerns a method for monitoring the development of a disease in a patient, wherein said disease is selected from the group consisting of
a) tissue trauma,
b) a reperfusion injury resulting from myocardial infarction or stroke, organ transplantations or an other surgical operation,
c) cancer or cancer metastasis, and
d) an inflammatory condition,
and wherein CD73 in a tissue fluid drawn from said patient is used as a biomarker, wherein the method is repeated at two or more points of time and an altered level of CD73 in the sample, compared to a previous assay, is used to indicate progression of the disease, or to indicate regression of the disease, wherein an increasing level of CD73 indicates regression of the disease and decreasing level of CD73 indicates progression of the disease.

### BRIEF DESCRIPTION OF THE DRAWINGS

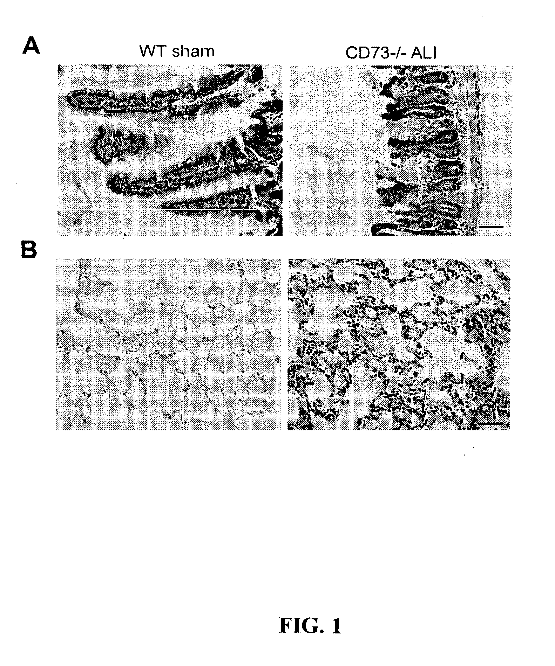

**Figure 1****.** The ischemia-reperfusion injury of the gut induces primary and secondary tissue damage. The mesenteric artery was occluded for 30 min and then allowed to be reperfused for 4 h. Representative micrographs from the (A) gut and (B) lungs of wild-type mice after sham operation (laparotomy only) and after induction of the IR-injury.

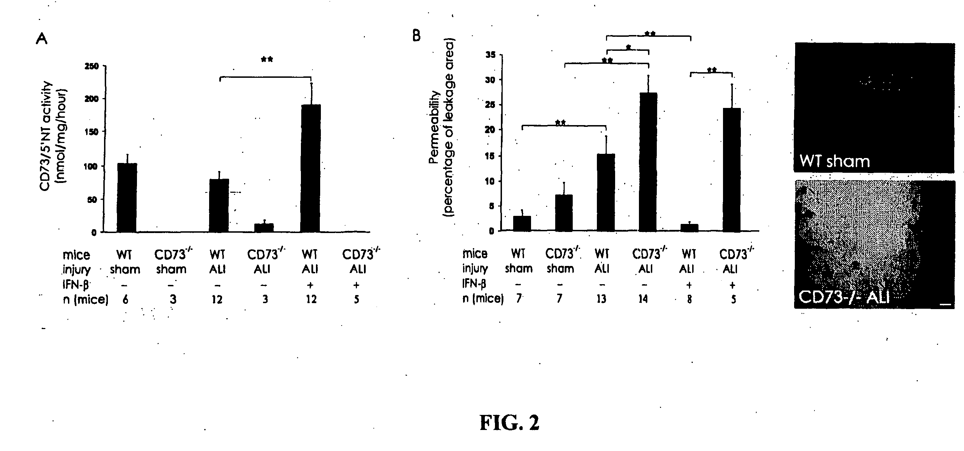

**Figure 2****.** Lung CD73/5'NT activity correlates inversely with the disease activity. CD73/5'NT^{-/-} mice and their wild-type (WT) littermates underwent sham operation (sham) or 30 minutes of intestinal ischemia followed by 240 minutes of reperfusion (ALI). In additional groups, animals exposed to ALI were pretreated with IFN-β. (A) Lung CD73/5'NT activity (mean±SEM, nmol of AMP hydrolyzed by milligram of protein per hour) measured from tissue lysates by TLC. (B) Semiquantitative analyses of vascular leakage (exudation of FITC-conjugated dextran) in lungs as measured from the histological sections using image analysis (% of section area exhibiting fluorescence above an arbitrarily chosen background value, mean±SEM). Representative micrographs from the indicated groups are also shown. Bar, 50 µm. * p<0.05, ** p<0.01.

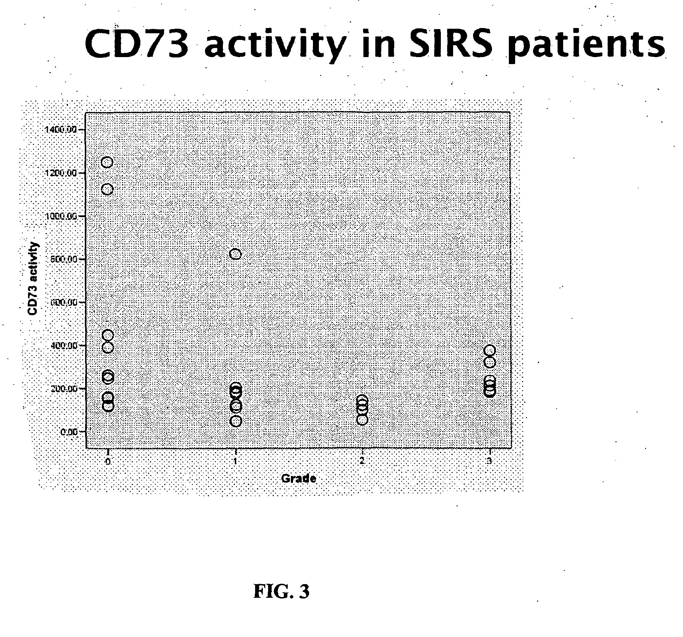

**Figure 3****.** CD73/5'NT activity in SIRS patients suffering from pancreatitis. The figure shows CD73/5'NT activity versus severity grade of pancreatitis: 0 = mild pancreatitis; 1 = severe pancreatitis without organ failure; 2 = severe pancreatitis with organ failure; 3 = healthy control.

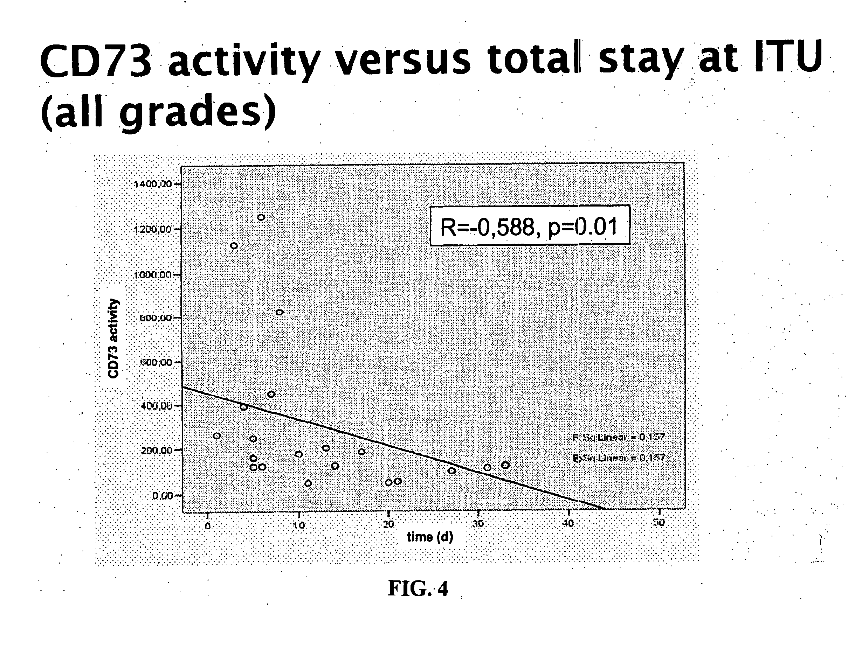

**Figure 4****.** CD73/5'NT activity versus total stay at intensive treatment unit (ITU) for patients with all grades of pancreatitis.

### DETAILED DESCRIPTION OF THE INVENTION

### Definitions and preferable embodiments:

The term "patient" or "individual" refers to a human or to an animal subject.

The term "monitoring the development of a disease" means that the progression of the disease (i.e. worsening of the disease) or the regression of the disease (i.e. a patient's recovery) can be made by comparing a measured level of the biomarker to a control or to one or more previous measurements, carried out at different points of time, of the level of the biomarker in the same patient. For example, a decreased level of the biomarker, compared to the result from a previous measurement or to a control may be used to indicate the progression of the disease, while an increased level of the biomarker, compared to the result from a previous measurement or to a control is used to indicate the regression of the disease. However, there are likely certain diseases affecting the level of the biomarker in the opposite way.

The term "tissue fluid" shall be understood to include any fluid which bathes and surrounds the cells. The term includes, for example, blood plasma, serum, lympha, urine, exudates (pleural, peritoneal) and cerebrospinal fluid.

The term "inflammatory condition" is meant to include any harmful and undesired inflammatory response in a tissue in an individual, wherein said inflammatory condition may result from an acute condition such as tissue trauma, a reperfusion injury resulting from myocardial infarction or stroke, organ transplantations or an other surgical operation, or from a chronic condition including allergic conditions, autoimmune diseases, and inflammatory diseases.

Diseases the development of which can be monitored by using CD73 protein in tissue fluid are typically selected from the group consisting of
a) tissue trauma,
b) a reperfusion injury resulting from myocardial infarction or stroke, organ transplantations or an other surgical operation,
c) cancer or cancer metastasis, and
d) an inflammatory condition.

Typical diseases leading to a change in the patient's CD73 level in tissue fluids, especially in serum are: tissue trauma; reperfusion injuries resulting from myocardial infarction or stroke, organ transplantations or other surgical operations; cancer or cancer metastasis; or inflammatory conditions resulting from the aforesaid traumas or reperfusion injuries or from chronic conditions including allergic conditions, autoimmune diseases, and inflammatory diseases. As examples of such chronic conditions can be mentioned arthritis, allergic conditions such as asthma, inflammatory conditions such as inflammatory bowel disease or an inflammatory condition of the skin, psoriasis, Parkinson's disease, Alzheimer's disease, autoimmune diseases, type I or type II diabetes, atherosclerosis, multiple sclerosis, Crohn's disease, or rejection reactions due to organ transplantations. Particularly, the inflammatory diseases systemic inflammatory response syndrome (SIRS), acute lung injury (ALI), multi-organ failure (MOF), ischemia reperfusion injury (IRI) and adverse drug reaction (ADRS) will lead to alterations of tissue fluid CD73 protein.

The term "cytokine" includes any protein or peptide used in organisms as signalling compounds. In particular, this term refers to an interferon or an interleukin, but is not restricted thereto. In case the cytokine is an interferon, the interferon may be alpha-, beta-, gamma-, omega-, or any other interferon and it can be any subtype of the aforementioned interferons. Interferons are used in the treatment of the aforementioned diseases. As examples of interleukins can be mentioned IL-4, IL-10, IL-13 and IL-20.

"Statins" form a class of hypolipidemic agents used to lower cholesterol levels in individuals, particularly to reduce the risk of cardiovascular diseases. Also inflammatory conditions, dementia, cancer, nuclear cataract and pulmonary hypertension may respond to treatment with statins.

Determining of CD73 protein in a sample drawn from an individual's tissue fluid can be carried out by an immunodetection by quantifying the level of the CD73 protein in said sample by subjecting the sample to a binder recognizing the CD73 protein, and quantifying said binder.

Alternatively, the detection can be carried out by detecting the activity of the CD73 protein in said sample by using thin layer chromatography or by subjecting said sample to a CD73 substrate, and monitoring the change of said substrate.

The term "binder" shall be understood to include antibodies, which can be monoclonal or polyclonal or genetically engineered; any antibody fragments; aptamers and affibodies, and any other binder capable of binding to an epitope on the CD73 protein. CD73 antibodies are well known in the art, see for example http://www.biocompare.com/matrixse/3194/6/67151/CD73.html. Affibodies represent a new kind of binders, small and especially stabile proteins, developed by Affibody Ab.

The binding assay can be competitive or non-competitive. One preferable assay is a sandwhich assay where a capture antibody (or other kind of binder) immobilized to a solid support, is subjected to the sample comprising the antigen, which at a first epitope binds to the capture antibody and adding a labeled antibody (or other kind of binder), directed to another epitope of the antigen. The labeled antibody is quantified either directly (homogeneous assay) or after separation of non-immobilized labeled antibodies. The label can be a radioactive isotope, a fluorescent dye, an enzyme or any other detectable label.

For example, for the purpose of immunodetection, any suitable anti-CD73 specific antibody can be used to capture soluble CD73 from the sample, and then the amount of bound protein can be quantified using a variety of techniques. For instance, a sandwhich ELISA can be employed in which one anti-CD73 antibody is immobilized to the bottom of multiwell plates, the sample is added, and the bound CD73 is detected using another anti-CD73 antibody. The anti-CD73 antibody is then detected using any of the multiple techniques suitable for antibody detection, such as labeled second-stage antibodies. The CD73 specificity of the reaction is controlled by including an irrelevant antibody as a capture or detection antibody and comparing the signals between these negative controls and anti-CD73 antibodies.

### Determining of CD73 activity:

CD73 activity can be measured using thin layer chromatography according to published protocols. CD73 activity can be also measured using any enzymatic assay that measures the conversion of AMP, or another purine mononucleotide that can be used as a CD73 substrate, into the corresponding nucleoside. For example, the assay can be based on conversion of radioactively or fluorescently labeled substrates. Detection methods can rely on the quantification of the decrease in a substrate concentration, or an increase in the product concentration or the release of the phosphate group. The CD73 dependence of the reaction can be determined by performing the assay in the presence and absence of a known CD73 inhibitor, such as AMPCP.

Suitable substrates for CD73 are, for example nucleoside-5'-monophosphates including adenosine-5'-monophosphate (AMP), inosine-5'-monophosphate (IMP), and the like.
The invention will be illuminated by the following non-restrictive Experimental Section.

### EXPERIMENTAL SECTION

### Materials and methods

### ALI model and vascular leakage

CD73^{-/-} mice, which were backcrossed to C57BL/6 background for 8 generations, and C57BL/6 wild-type (WT) mice were used. They lack CD73 mRNA, protein and enzyme activity [3]. The animals were weight-, sex- and age-matched. All mice had access to standard mouse chow and water until the experiment.
Mice were anesthesized with ketamine hydrochloride (100 mg/kg of body weight, i.p.) and xylazine (10 mg/kg of body weight, i.p.). During the anesthesia the mice spontaneously ventilated normal air. Before surgery animals received 1 ml of sterile saline subcutaneously to compensate for peroperative fluid loss. Superior mesenteric artery was dissected via midline laparotomy and occluded by microvascular clamp for 30 minutes. Sham animals underwent superior mesenteric artery dissection without vascular occlusion. The wound was sutured in one layer. The body temperature of the animals was maintained throughout the ischemia phase with a heating lamp. After the ischemia, the microvascular clamp was released, the wound sutured and animals received additional 1 ml of saline subcutaneously. After 235 minutes of reperfusion mice received FITC-conjugated dextran (25 mg/kg body weight in 0.2 ml sterile saline; mw. 70 000 D, Molecular Probes). Mice were sacrificed after 240 minutes of reperfusion and the tissue samples were collected. The 30 min ischemia-240 min reperfusion protocol is an established and reproducible ALI model [9]. The protocol was approved by the Committee on Animal Ethics of Turku University (permission n:o 1597/05 to Sirpa Jalkanen).

The effect of IFN-β on CD73 activity and permeability was studied using pre- and post-treatment protocols. Subgroups of mice were pretreated with recombinant mouse IFN-β (6000 IU s.c. once daily for 3 days prior to ischemia). In the post-treatment group, the animals got a single bolus of IFN-β (20 000 IU) intravenously after the ischemic phase at the beginning of the reperfusion period.

All mice were injected i.v. with FITC-conjugated dextran (70 kDa) 5 minutes before euthanasia. Vascular leakage was determined from three color images taken from randomly chosen fields from cryosectioned lungs using computational image analysis (Image J).

### Analyses of CD73 activity

Ecto-5'-nucleotidase activity was assayed by TLC, as described previously [10]. Briefly, the standard enzyme assay contained in a final volume of 120 µl of RPMI 1640, lung lysate, 5 mmol/L β-glycerophosphate, and the indicated concentrations of AMP with tracer [2-³H]AMP (sp. act., 18.6 Ci/mmol; Amersham, Little Chalfont, U.K.). Incubation times were chosen to ensure the linearity of the reaction with time, so that the amount of the converted AMP did not exceed 7-10% of the initially introduced substrate. Aliquots of the mixture were applied to Alugram SIL G/UV₂₅₄ TLC sheets (Macherey-Nagel, Düren, Germany) and separated with isobutanol/isoamyl alcohol/2-ethoxyethanol/ammonia/H₂0 (9:6:18:9:15) as solvent. ³H-labeled AMP and its dephosphorylated nucleoside derivatives were visualized in UV light and quantified using a Wallac-1409β- spectrometer. CD73 activity was expressed as nM of AMP hydrolyzed by milligram of protein per hour. Protein concentration in the lysates was determined by BCA Protein Assay Kit (Pierce, Rockford, IL) according to manufacturer's instructions.

### Statistical assays

Non-parametric one-way ANOVA (Kruskall-Wallis and Mann-Whitney U tests) were used.

### Results

### CD73 activity correlates with the disease activity

Intestinal ischemia-reperfusion (IR) caused marked tissue damage both in the gut and in the lungs (Fig. 1). In the lungs of the sham-operated wild-type mice the CD73 activity was low (Fig. 2A). The microscopic analyses of FITC-dextran in the lungs showed that there was only marginal leakage in WT mice undergoing sham operation (Fig. 2B).

When ALI was induced to wt animals, the CD73 activity was reduced by 25%. At the same time vascular leakage increased significantly. Changes in vascular permeability directly correlate to the disease severity, since the leakage of intravascular fluids to the lung parencyma and further to the alveoli is the major cause for deteriorating lung function and impaired gas exchange.

As expected, the CD73 activity was undetectable or at extremely low levels in CD73 deficient mice both after sham operation and after intestinal IR. In CD73 deficient mice the leakage in sham-operated animals was mildly increased when compared to the wild-type sham operated mice. Notably, when ALI was induced CD73 deficient mice showed about 80% more leakage in the lungs than their WT littermates (p=0.03).

These data show that there is an inverse correlation between the CD73 activity and vascular permeability, a measure of disease severity.

### CD73 activity correlates with the treatment response

IFN-β pretreatment for 3 days (at a dose clinically used in the treatment of multiple sclerosis) led to a 230 % increase in CD73 activity in WT lungs during ALI (p=0.002, Fig. 2A). Most strikingly, the leakage area in WT mice after induction of ALI was reduced by more than 90% after the IFN-β pretreatment when compared to non-treated littermates (p=0.0001, Fig. 2B). In fact, it was not different from animals undergoing only the sham operation. Strikingly, IFN-β also had no protective effects on ALI in CD73 deficient mice. These data show that interferon beta treatment decreases vascular leakage in a strictly CD73 dependent manner. Moreover, increase in CD73 activity in wild-type mice can be used to predict beneficial outcome in response to IFN-β treatment.

We then tested whether IFN-β treatment could reverse an already established capillary injury. To that end we treated the mice with IFN-β only after the ischemic period at the time of reperfusion. Notably, the single IFN-β dose highly significantly improved vascular barrier function during the following 4 h reperfusion period. The leakage of FITC dextran was reduced by 90±9 % in the post-treatment group when compared to the controls (n=8-13 mice/group, p<0.001). At the same time, CD73 activity measure from serum samples increased by more than 30% (from 427±22 in ALI group without treatment to 561±48 in ALI group treated with IFN-β; p=0.04, n=4/group). Thus, the induction of CD73 activity correlates positively with a treatment response. Moreover, measuring CD73 activity in a blood sample can yield useful information about the IFN-β responsiveness.

### References

1 Hasko, G. and Cronstein, B. N., Adenosine: an endogenous regulator of innate immunity. Trends Immunol. 2004. 25: 33-39.
2 Ohta, A. and Sitkovsky, M., Role of G-protein-coupled adenosine receptors in downregulation of inflammation and protection from tissue damage. Nature 2001. 414: 916-920.
3 Thompson, L. F., Eltzschig, H. K., Ibla, J. C., Van De Wiele, C. J., Resta, R., Morote-Garcia, J. C. and Colgan, S. P., Crucial role for ecto-5'-nucleotidase (CD73) in vascular leakage during hypoxia. J. Exp. Med. 2004. 200: 1395-1405.
4 Lennon, P. F., Taylor, C. T., Stahl, G. L. and Colgan, S. P., Neutrophil-derived 5'-adenosine monophosphate promotes endothelial barrier function via CD73-mediated conversion to adenosine and endothelial A2B receptor activation. J. Exp. Med. 1998. 188: 1433-1443.
5 Synnestvedt, K., Furuta, G. T., Comerford, K. M., Louis, N., Karhausen, J., Eltzschig, H. K., Hansen, K. R., Thompson, L. F. and Colgan, S. P., Ecto-5'-nucleotidase (CD73) regulation by hypoxia-inducible factor-1 mediates permeability changes in intestinal epithelia. J. Clin. Invest. 2002. 110: 993-1002.
6 Henttinen, T., Jalkanen, S. and Yegutkin, G. G., Adherent leukocytes prevent adenosine formation and impair endothelial barrier function by Ecto-5'-nucleotidase/CD73-dependent mechanism. J. Biol. Chem. 2003. 278: 24888-24895.
7 Eckle, T., Fullbier, L., Wehrmann, M., Khoury, J., Mittelbronn, M., Ibla, J., Rosenberger, P. and Eltzschig, H. K., Identification of Ectonucleotidases CD39 and CD73 in Innate Protection during Acute Lung Injury. J. Immunol. 2007. 178: 8127-8137.
8 Niemela, J., Henttinen, T., Yegutkin, G. G., Airas, L., Kujari, A. M., Rajala, P. and Jalkanen, S., IFN-alpha induced adenosine production on the endothelium: a mechanism mediated by CD73 (ecto-5'-nucleotidase) up-regulation. J. Immunol. 2004. 172: 1646-1653.
9 Ohara, M., Unno, N., Mitsuoka, H., Kaneko, H. and Nakamura, S., Peritoneal lavage with oxygenated perfluorochemical preserves intestinal mucosal barrier function after ischemia-reperfusion and ameliorates lung injury. Crit. Care Med. 2001. 29: 782-788.
10 Yegutkin, G. G., Henttinen, T. and Jalkanen, S., Extracellular ATP formation on vascular endothelial cells is mediated by ecto-nucleotide kinase activities via phosphotransfer reactions. Faseb J. 2001. 15: 251-260.
11 Airas, L., Niemelä, J., Yegutkin, G. and Jalkanen, S., Mechanism of Action of IFN-β in the Treatment of Multiple Sclerosis. Ann N.Y. Acad. Sci. 1110:641-648 (2007).
12 S. M. Johnson, S. Patel, F. E. Bruckner and D. A. Collins, 5'- Nucleotidase as a marker of both general and local inflammation in rheumatoid arthritis patients. Rheumatology, 1999; 38: 391-396

## Claims

1. A method for monitoring the development of a disease in a patient, wherein said disease is selected from the group consisting of
a) tissue trauma,
b) a reperfusion injury resulting from myocardial infarction or stroke, organ transplantations or an other surgical operation,
c) cancer or cancer metastasis, and
d) an inflammatory condition,
and wherein CD73 in a tissue fluid drawn from said patient is used as a biomarker, wherein the method is repeated at two or more points of time and an altered level of CD73 in the sample, compared to a previous assay, is used to indicate progression of the disease, or to indicate regression of the disease, wherein an increasing level of CD73 indicates regression of the disease and decreasing level of CD73 indicates progression of the disease.

2. The method according to claim 1 where the disease is an inflammatory disease.

3. The method according to claim 2 where the inflammatory disease is systemic inflammatory response syndrome (SIRS), acute lung injury (ALI), multi-organ failure (MOF), ischemia reperfusion injury (IRI) or adverse drug reaction (ADRS).

## Patentansprüche

1. Verfahren zur Überwachung der Entwicklung einer Krankheit bei einem Patienten, wobei die Krankheit aus der Gruppe, bestehend aus
a) Gewebetrauma,
b) einer Reperfusionsschädigung, resultierend aus Myokardinfarkt oder Schlaganfall, Organtransplantationen oder einem anderen chirurgischen Eingriff,
c) Krebs oder Krebsmetastase und
d) einem Entzündungszustand, ausgewählt ist,
und wobei CD73 in einem Gewebefluid, das dem Patienten entnommen wurde, als Biomarker verwendet wird, wobei das Verfahren zu zwei oder mehr Zeitpunkten wiederholt wird und ein veränderter Level an CD73 in der Probe im Vergleich zu einem vorherigen Assay verwendet wird, um eine Progression der Krankheit anzuzeigen oder um eine Regression der Krankheit anzuzeigen, wobei ein steigender Level an CD73 eine Regression der Krankheit anzeigt und ein sinkender Level an CD73 eine Progression der Krankheit anzeigt.

2. Verfahren gemäß Anspruch 1, wobei die Krankheit eine Entzündungskrankheit ist.

3. Verfahren gemäß Anspruch 2, wobei die Entzündungskrankheit systemisches inflammatorisches Response-Syndrom (SIRS), akute Lungeninsuffizienz (ALI), Multiorganversagen (MOF), Ischämie-Reperfusionsschädigung (IRI) oder Arzneimittelnebenwirkung (adverse drug reaction (ADRS)) ist.

## Revendications

1. Procédé de surveillance de l'évolution d'une maladie chez un patient, dans lequel ladite maladie est choisie dans le groupe formé par :
a) un traumatisme tissulaire,
b) une lésion de reperfusion consécutive à un infarctus du myocarde ou à un accident vasculaire, à des transplantations d'organe ou à une autre intervention chirurgicale,
c) un cancer ou des métastases cancéreuses, et
d) une affection inflammatoire,
et dans lequel CD73, dans un fluide tissulaire prélevé sur ledit patient, est utilisé en tant que biomarqueur, dans lequel le procédé est répété à deux instants ou plus et une altération du niveau de CD73 dans le prélèvement, par rapport à un dosage précédent, est utilisée pour indiquer une progression de la maladie ou pour indiquer une régression de la maladie, dans lequel une augmentation du niveau de CD73 indique une régression de la maladie et une diminution du niveau de CD73 indique une progression de la maladie.

2. Procédé selon la revendication 1, dans lequel la maladie est une maladie inflammatoire.

3. Procédé selon la revendication 2, dans lequel la maladie inflammatoire est un syndrome de réponse inflammatoire systémique (SIRS), une lésion pulmonaire aiguë (ALI), une défaillance multiviscérale (MOF), une lésion d'ischémie-reperfusion (IRI) ou une réaction médicamenteuse indésirable (ADR).
